# EUROPÄISCHE PATENTSCHRIFT

(11) **EP 3 854 357 B1**
(45) Veröffentlichungstag und Bekanntmachung des Hinweises auf die Patenterteilung: **03.05.2023**
(21) Anmeldenummer: 21150860.1
(22) Anmeldetag: 11.01.2021
(51) Int. Cl.: A61F 5/01, A61F 5/30

(54) **KNIEGELENKBANDAGE**
KNEE JOINT BANDAGE
BANDAGE POUR ARTICULATION DE GENOU

(30) Priorität: 24.01.2020 DE 102020101745
(43) Veröffentlichungstag der Anmeldung: 28.07.2021
(73) Patentinhaber: Julius Zorn GmbH, 86551 Aichach (DE)
(72) Erfinder: FISCHER, Markus, 80687 München (DE); BERG, Roland, 12527 Berlin (DE)
(74) Vertreter: Charrier Rapp & Liebau

(56) Entgegenhaltungen:
- EP-A1- 2 954 879
- EP-A2- 3 090 709
- EP-B1- 2 954 879
- EP-B1- 3 090 709
- DE-A1-102013 022 088
- DE-U1- 29 519 979
- DE-U1- 29 803 103
- FR-A1- 2 607 384
- FR-A1- 2 807 644

## Beschreibung

Die Erfindung betrifft eine Kniegelenkbandage nach dem Oberbegriff des Anspruchs 1.

Aus dem Stand der Technik sind Orthesen in Form von textilen Bandagen zur Korrektur und/oder Entlastung des Kniegelenks und insbesondere zur Beeinflussung des Patellagleitwegs bekannt. Diese Orthesen umfassen einen schlauchförmigen Grundkörper aus einem Textilmaterial und weisen mindestens eine, lokal um die Patella angeordnete Druckpelotte aus einem flexiblen Material, beispielsweise aus Silikon, auf. Die wenigstens eine Pelotte gibt der Patella bei Bewegungen des Kniegelenks eine Führung und kann dadurch bei Schädigung des Kniegelenks im Gleitlager zwischen der Patella und dem Oberschenkel zu einer Schmerzreduktion beitragen. Durch einstellbare Zug- oder Druckelemente, wie z.B. Zugbänder, die mit der Pelotte zugfest verbunden sind, soll eine positive Beeinflussung des Patellagleitwegs bei einer Bewegung des Kniegelenks in einer physiologisch günstigen Position erreicht werden.

Eine derartige Orthese in Form einer Bandage mit einem elastischen Textilstrumpf und einer in oder an dem Textilstrumpf angeordneten Pelotte in Form einer elastischen Profileinlage ist aus der EP 2 590 603 B1 bekannt, wobei die Pelotte ring- oder spangenförmig ausgebildet ist und zumindest den lateralen, wadenbeinseitigen Abschnitt der Patella des Kniegelenks umgreift, wenn die Bandage an einem Kniegelenk angelegt ist. Die Bandage enthält ein sich im Wesentlichen in Längsrichtung der Bandage über das Kniegelenk hinweg erstreckendes, flexibles aber nicht dehnbares Zugband, das lateral in oder entlang der Pelotte geführt ist und an Verankerungspunkte am unteren und oberen Ende des Zugbands an dem Textilstrumpf befestigt ist. Beim Beugen des Knies wird durch ein Auseinanderwandern der Verankerungspunkte eine die Patella zentrierende Kraft ausgeübt. Dabei ist die Wirklänge des Zugbands an zumindest einem Ende veränderbar, indem der Verankerungspunkt dieses Endes des Zugbands veränderlich ausgebildet ist. Durch Einstellung der Wirklänge des Zugbands kann die vom Zugband ausgeübte Kraft, welche die Patella zentrieren und in eine physiologische Normallage verschieben soll, gesteuert werden. Wenn auf mindestens ein Ende des an den Verankerungspunkten verankerten Zugbands eine Zugkraft ausgeübt wird, die bei angelegter Bandage insbesondere beim Beugen des Knies durch das Auseinanderwandern der Verankerungspunkte erzeugt wird, wirkt diese Zugkraft verschiebend bzw. fixierend auf die Patella, so dass insbesondere bei einem Kniedefekt einer im Kniegelenk auf die Patella wirkenden dislozierenden Kraft durch das Zugband eine entsprechend angepasste Gegenkraft entgegengesetzt wird, die der Verschiebung der Patella in eine physiologisch ungünstige Position entgegenwirkt.

Diese bekannte Bandage führt bei einer Bewegung des Kniegelenks, insbesondere beim Beugen des Knies, zu einer lateral auf die Patella einwirkenden Kraft, die ein Abdriften der Patella nach außen, also in lateraler Richtung, unterdrückt. Die bekannte Bandage ist jedoch nicht geeignet, eine Bewegung der Patella nach oben, also in proximaler Richtung, zu verhindern.

Aus der DE 295 19 979 U1 ist eine Kniebandage bekannt, die einen schlauchförmigen Grundkörper aus einem elastischen Textilmaterial, zumindest eine am Grundkörper angeordnete Pelotte und ein mit dieser Pelotte verbundenes Zugband mit einem ersten und einem zweiten Ende umfasst, wobei die Pelotte bei an einem Kniegelenk angelegter Kniegelenkbandage proximal zur Patella positioniert ist und die Patella teilweise in ihrem proximalen Bereich umgreift und die beiden Enden des Zugbands distal der Patella am Grundkörper mittels lösbarer Fixiermittel verankert sind.

Weitere Kniegelenkbandagen mit einer die Patella zumindest teilweise umgreifenden Pelotte sind aus der EP 3090 709 A2, der FR 2 607 384 A1 und der FR 2 807 644 A1 bekannt.

Durch eine angeborene Anomalie oder infolge einer Patellasehnenruptur oder einer Patellaluxation kann es zu einem Patellahochstand kommen. Ein Hochstand der Patella kann sich auch bei der Osgood-Schlatter-Krankheit einstellen. Die oben beschriebene, aus dem Stand der Technik bekannte Bandage ist nicht für eine Therapie eines Patellahochstands geeignet.

Aus dem Stand der Technik, wie z.B. der EP 2 954 879 B1 sind weiterhin Kniegelenkbandagen bekannt, die über wenigstens eine Pelotte verfügen, die quer über die Patellasehne verläuft, um durch gezielt auf die Patellasehne ausgeübten Druck durch Entzündungen der Patellasehne hervorgerufene Schmerzen zu lindern, wie sie beispielsweise beim Patellasehnenspitzensyndrom auftreten können. Derartige Patellasehnenbandagen mit einer quer über die Patellasehne verlaufenden Pelotte, die zu einer Vorspannung der Patellasehne sowie zu einer Stabilisierung des Kniegelenks und dadurch zu einer Schmerzlinderung beiträgt, kann jedoch bei einer Bewegung des Kniegelenks ein Hochschieben der Patella in eine physiologisch ungünstige Position bewirken.

Hiervon ausgehend besteht ein Bedürfnis für eine Kniegelenkbandage, die einerseits eine laterale Stabilisierung der Patella bewirkt und andererseits einem Patellahochstand und/oder einem Hochschieben der Patella bei einer Bewegung des Kniegelenks entgegenwirkt.

Der Erfindung liegt daher die Aufgabe zugrunde, eine Kniegelenkbandage bereit zu stellen, die bei einer Therapie von Kniegelenksbeschwerden eine optimierte Bewegungsführung des Kniegelenks ermöglicht und insbesondere einerseits ein Abdriften der Patella nach außen (also in lateraler Richtung) und andererseits ein Hochschieben der Patella in proximaler Richtung unterdrückt. Insbesondere soll die Kniegelenkbandage bei der Therapie von Kniegelenksbeschwerden eine sowohl in medial-lateraler Richtung als auch in proximaldistaler Richtung eine zentrierende Wirkung auf die Patella ausüben, um diese bei einer Bewegung des Kniegelenks in einer physiologisch optimierten Position zu halten. Weiterhin soll die Kniegelenkbandage eine individuelle Anpassung an die Anatomie eines Patienten ermöglichen und eine Durchblutung des das Kniegelenk umgebenden Gewebes fördern sowie Schwellungen und Ergüsse abbauen und Reizungen und Schmerzen lindern.

Diese Aufgaben werden mit einer Kniegelenkbandage mit den Merkmalen des Anspruchs 1 gelöst. Bevorzugte Ausführungsformen dieser Kniegelenkbandage sind den abhängigen Ansprüchen zu entnehmen.

Die Kniegelenkbandage gemäß der Erfindung umfasst einen schlauchförmigen Grundkörper aus einem elastischen Textilmaterial, zumindest eine am Grundkörper angeordnete erste Pelotte und ein mit dieser Pelotte verbundenes Zugband mit einem ersten und einem zweiten Ende, wobei die erste Pelotte bei an einem Kniegelenk angelegter Kniegelenkbandage proximal zur Patella positioniert ist und die Patella zumindest teilweise, insbesondere im proximalen Bereich, umgreift und die beiden Enden des Zugbands distal der Patella am Grundkörper mittels lösbarer Fixiermittel verankerbar sind. Um eine bei einer Bewegung des Kniegelenks stabilisierende und die Patella zentrierende Kraft sowohl in medialer als auch in distaler Richtung auf die Patella auszuüben, ist erfindungsgemäß das erste Ende des Zugbands an einem vorgegebenen ersten Verankerungspunkt und das zweite Ende des Zugbands an einem vorgegebenen zweiten Verankerungspunkt am Grundkörper der Bandage verankerbar, wobei bei angelegter Bandage beide Verankerungspunkte jeweils distal der Patella liegen und gleichzeitig entweder beide Verankerungspunkte medial zur Patellasehne liegen oder einer der beiden Verankerungspunkte medial zur Patellasehne und der andere Verankerungspunkt distal zur Patellasehne in der Linie der Verlängerung der Patellasehne in Längsrichtung auf dem Schienbein liegt.

Anders als bei den aus dem Stand der Technik bekannten Kniebandagen oder -orthesen, liegen die Verankerungspunkte der beiden Enden des Zugbands damit einerseits distal der Patella und andererseits entweder beide medial zur Patellasehne oder es liegt einer der beiden Verankerungspunkte medial zur Patellasehne während der andere Verankerungspunkt distal zur Patellasehne in der Linie der Verlängerung der Patellasehne in Längsrichtung auf dem Schienbein liegt. In Bezug auf eine durch das Kniegelenk verlaufende Sagittalebene liegen die beiden Verankerungspunkte der beiden Enden des Zugbands also nicht symmetrisch um die Sagittalebene, sondern es liegen entweder beide Verankerungspunkte distal dieser Sagittalebene oder es liegt nur einer der beiden Verankerungspunkte medial zur Sagittalebene und der andere Verankerungspunkt liegt distal zur Patellasehne in dieser Sagittalebene, die durch die Verlängerung der Patellasehne in deren Längsrichtung auf dem Schienbein definiert ist.

Durch diese Anordnung der Verankerungspunkte, an denen die beiden Enden des Zugbands am Grundkörper der Kniegelenkbandage verankert werden, wird bei einer Bewegung des Kniegelenks, insbesondere beim Beugen des Knies, durch die erste Pelotte, die zugfest mit dem Zugband verbunden ist, eine Kraft auf die Patella ausgeübt, die einerseits in medialer und andererseits in distaler Richtung auf die Patella einwirkt und dadurch verhindert, dass die Patella nach außen oder nach oben in eine physiologisch ungünstige Stellung verschoben wird. Die erfindungsgemäße Kniegelenkbandage bewirkt daher einerseits eine Lateralisierung der Patella und wirkt andererseits einem Hochschieben der Patella oder einem Patellahochstand entgegen.

Dieser Effekt wird dabei durch ein Zusammenwirken der aus einem elastischen Material, wie z.B. Silikon, gefertigten ersten Pelotte und der zugfest mit dieser Pelotte verbundenen Zugband sowie dessen Verankerung an den definierten Verankerungspunkten (erster und zweiter Verankerungspunkt) bewirkt. Die erste Pelotte ist dabei bevorzugt U-förmig oder hufeisenförmig ausgebildet, wobei die erste Pelotte bei angelegter Kniegelenkbandage zumindest den proximalen Bereich der Patella umgreift. Zur Erzielung eines Massageeffekts, der insbesondere eine durchblutungsfördernde Wirkung erzeugt, kann die erste Pelotte über Stimulationselemente verfügen, wie z.B. über eine im medial-proximalen Bereich der ersten Pelotte angeordnete Ausbuchtung, die eine stimulierende Wirkung auf die Muskulatur ausübt. Die erste Pelotte kann auch über Stimulationselemente in Form von Noppen oder Rippen verfügen, welche eine massierende Wirkung auf das die Patella umgebende Gewebe ausüben.

Das Textilmaterial des Grundkörpers ist zweckmäßig elastisch ausgebildet. Insbesondere kann der Grundkörper aus einem elastischen Gestrick gebildet sein. Durch die Elastizität des schlauchförmigen Grundkörpers wird bei angelegter Kniegelenkbandage eine Kompression auf die das Kniegelenk umgebende Muskulatur ausgeübt, wodurch ein Massageeffekt und eine durchblutungsfördernde Wirkung erzeugt wird. Durch die von dem elastischen Textilmaterial des schlauchförmigen Grundkörpers ausgeübte Kompression werden weiterhin Schwellungen und Ergüsse im Bereich des Kniegelenks abgebaut.

Das Zugband ist zweckmäßig unelastisch oder zumindest deutlich weniger elastisch als das Material der ersten Pelotte. Das Zugband kann entweder durchgehend vom ersten Ende zum zweiten Ende ausgebildet sein und abschnittsweise zugfest mit der ersten Pelotte verbunden sein. Insbesondere kann das durchgehend ausgebildete Zugband im Bereich der ersten Pelotte durch das elastische Material der ersten Pelotte durchlaufen und dabei mit der ersten Pelotte verbunden sein, indem das Zugband in das elastische Material der ersten Pelotte, bei dem es sich beispielsweise um Silikon oder Silikonkautschuk handeln kann, eingegossen ist. Das Zugband kann jedoch auch abschnittsweise an Unterbrechungsstellen unterbrochen sein, wobei das abschnittsweise unterbrochene Zugband an den Unterbrechungsstellen mit der ersten Pelotte zugfest verbunden ist, indem das Zugband an den Unterbrechungsstellen beispielsweise mit dem elastischen Material der ersten Pelotte verklebt ist.

In einem bevorzugten Ausführungsbeispiel der Erfindung sind die Enden des Zugbands mittels Fixiermittel an der Außenseite des Grundkörpers verankerbar. Zweckmäßig umfassen die Fixiermittel dabei lösbare Befestigungsmittel, wie z.B. Klettverschlüsse, wobei bevorzugt an jedem Ende des Zugbands ein solches Befestigungsmittel angeordnet ist, mit dem das jeweiligen Ende des Zugbands lösbar an der Außenseite des Grundkörpers befestigt werden kann. Wenn die (freien) Enden des Zugbands mittels der Befestigungsmittel an der Außenseite des Grundkörpers befestigt sind, sind die beiden Verankerungspunkte, also der erste Verankerungspunkt des ersten Endes und der zweite Verankerungspunkt des zweiten Endes, durch die Position der Befestigungsmittel definiert, über welche das jeweilige Ende des Zugbands an der Außenseite des Grundkörpers befestigt ist.

In einer weiteren Ausführungsform der Erfindung umfassen die Fixiermittel für jedes Ende des Zugbands Umlenkmittel, welche das Zugband nahe des jeweiligen Endes umlenken. Wie bei dem ersten Ausführungsbeispiel sind auch bei diesem Ausführungsbeispiel an jedem Ende des Zugbands Befestigungsmittel angeordnet, mit dem das jeweilige Ende des Zugbands lösbar an einem Abschnitt des Zugbands befestigbar ist. Dieses Ausführungsbeispiel ermöglicht die Ausübung höherer Zugkräfte aufgrund eines durch die Umlenkung des Zugbands an seinen Enden bewirkten Flaschenzugeffekts. Bei diesem Ausführungsbeispiel sind die beiden Verankerungspunkte, also der erste Verankerungspunkt des ersten Endes des Zugbands und der zweite Verankerungspunkt des zweiten Endes des Zugbands, durch die Stellen definiert, an denen das Zugband im Bereich des ersten Endes und im Bereich des zweiten Endes durch die Umlenkmittel umgelenkt wird. Die Position der Umlenkmittel, bei denen es sich beispielsweise um Umlenkösen oder -öffnungen in einer Umlenklasche handeln kann, definiert dabei die beiden Verankerungspunkte.

Die erste Pelotte ist bevorzugt in dem Grundkörper der Kniegelenkbandage integriert. Dadurch ist eine sichere und positionsgerechte Anordnung der ersten Pelotte um die Patella beim Anlegen der Kniegelenkbandage gewährleistet. Die erste Pelotte kann dabei zweckmäßig in einer Tasche geordnet sein, die an der Innenseite des Grundkörpers vorgesehen und beispielsweise durch Vernähen oder Verstricken mit dem Grundkörper befestigt ist. Im Bereich der Tasche sind dabei zweckmäßig zwei Öffnungen in dem Grundkörper vorgesehen. Die Öffnungen können insbesondere als schmale Schlitze ausgebildet sein, die sich in lateral-medialer Richtung (also etwa horizontal) erstrecken und/oder schräg zur lateral-medialer Richtung verlaufen können. Durch diese Öffnungen können Abschnitte des Zugbands vom Inneren des schlauchförmigen Grundkörpers nach außen durchgeführt werden.

Das Zugband kann dabei aus mehreren Abschnitten zusammengesetzt sein und insbesondere einen ersten und einen zweiten Abschnitt umfassen, wobei der erste Abschnitt das erste Ende des Zugbands und der zweite Abschnitt das zweite Ende des Zugbands enthält und der erste und der zweite Abschnitt des Zugbands auf der Außenseite des schlauchförmigen Grundkörpers angeordnet sind. Der erste und der zweite Abschnitt des Zugbands sind dabei durch einen im Inneren des schlauchförmigen Grundkörpers und insbesondere durch die erste Pelotte verlaufenden dritten Abschnitt miteinander verbunden, so dass sich ein durchgehendes Zugband aus den drei Abschnitten zusammensetzt, welches bereichsweise innerhalb und bereichsweise außerhalb des schlauchförmigen Grundkörpers verläuft und durch die Öffnungen in dem Grundkörper von innen nach außen geführt ist. Der erste und der zweite Abschnitt des Zugbands liegt dadurch auf der Außenseite des schlauchförmigen Grundkörpers, weshalb der erste und der zweite Abschnitt im Folgenden auch als äußere Abschnitte bezeichnet werden.

Die Position der Öffnungen, durch die die äußeren Abschnitte des Zugbands nach außen geführt sind, bestimmt dabei zusammen mit der Position der Verankerungspunkte die Lage der äußeren Abschnitte des Zugbands, die sich jeweils zwischen der zugeordneten Öffnung und dem jeweiligen Verankerungspunkt (erster bzw. zweiter Verankerungspunkt) erstrecken.

Durch die Anordnung der Verankerungspunkte in der erfindungsgemäßen Kniegelenkbandage verläuft einer der beiden äußeren Abschnitte (zweiter Abschnitt) dabei zumindest im Wesentlichen in distal-proximaler Richtung, während der andere äußere Abschnitt (erster Abschnitt) schräg dazu verläuft, insbesondere von proximal-lateral nach distal-medial. In einer bevorzugten Ausführungsform schließen die beiden äußeren Abschnitte (erster und zweiter Abschnitt) des Zugbands einen Winkel im Bereich von 30° bis 60°, insbesondere von 45° ein. Durch diese Anordnung der beiden äußeren Abschnitte (erster und zweiter Abschnitt) des Zugbands erfolgt bei einer Bewegung des Knies eine Kraftausübung auf die Patella mit einer Kraftrichtung, die einerseits eine Komponente in distaler Richtung und andererseits eine Komponente in medialer Richtung aufweist.

Die vom Zugband auf die erste Pelotte bei einer Bewegung des Kniegelenks aktiv ausgeübte Kraft kann dabei durch die Positionen bestimmt werden, an denen die beiden Enden des Zugbands entweder an der Außenseite des Grundkörpers oder - bei umgelegten Bandabschnitten - auf einem Abschnitt des jeweiligen Bands mittels der Befestigungsmittel befestigt werden. Dadurch können unterschiedlich hohe Kräfte ausgeübt werden, je nach Position der fixierten Enden des Zugbands. Die ausgewählte Zugkraft kann dabei je nach Art oder Ziel der Therapie oder dem Grad der zu therapierenden Beschwerden angepasst werden. Die Richtung der ausgeübten Kräfte wird durch die Lage des Zugbands, insbesondere der äußeren Abschnitte des Zugbands, bestimmt.

Die erfindungsgemäße Kniegelenkbandage kann neben der ersten Pelotte, die zweckmäßig in dem schlauchförmigen Grundkörper integriert und insbesondere in einer Tasche angeordnet ist, noch eine zweite Pelotte umfassen, wobei die zweite Pelotte bei angelegter Kniegelenkbandage quer über die Patellasehne verläuft und distal zur ersten Pelotte bzw. distal zur Patella angeordnet ist. Diese zweite Pelotte übt bei angelegter Kniegelenkbandage einen dosierten Druck auf die Patellasehne, insbesondere an ihrem Ansatz, aus und kann dadurch die Patellasehne entlasten. Weiterhin führt diese zweite Pelotte zu einer Vorspannung der Patellasehne, wodurch bei einer Bewegung des Kniegelenks eine weitere Stabilisierung erfolgt. Die zweite Pelotte wirkt also wie eine Patellasehnenbandage, welche Patellasehnenreizungen therapieren und dadurch hervorgerufene Schmerzen lindern kann.

Bevorzugt ist die zweite Pelotte abnehmbar an der dem Kniegelenk zugewandten Innenseite des Grundkörpers befestigt, beispielsweise mittels lösbarer Befestigungsmittel, wie z.B. Klettverschlüsse. Durch die lösbare Anordnung der zweiten Pelotte an der Innenseite des Grundkörpers der Kniegelenkbandage kann diese in unterschiedlichen Therapiephasen bei der Therapie von Kniegelenkbeschwerden eingesetzt werden. Insbesondere kann in einer ersten Therapiephase eine Therapie des Kniegelenks sowohl mit der ersten als auch mit der zweiten Pelotte erfolgen, in dem die zweite Pelotte an der Innenseite des Grundkörpers befestigt und die Kniegelenkbandage in diesem Zustand an einem Kniegelenk angelegt wird. In dieser ersten Therapiephase verhindert die erste Pelotte in Verbindung mit dem an den Verankerungspunkten verankerten Zugband ein Hochschieben der Patella durch die distal der Patella angelegte und quer über die Patellasehne verlaufende zweite Pelotte bei einer Bewegung des Kniegelenks, insbesondere bei einer Beugung des Knies.

In einer zweiten Therapiephase kann die zweite Pelotte von der Kniegelenkbandage gelöst und in Verbindung mit einem Halteband am Kniegelenk unterhalb (also distal) der Patella angeordnet werden. In dieser zweiten Therapiephase wirkt die zweite Pelotte zusammen mit dem Halteband als Patellasehnenbandage, welche einen Druck auf die Patellesehne ausübt und dadurch zu einer Vorspannung der Patellasehne führt, wodurch Patellasehnenschmerzen verringert werden können. Die zweite Therapiephase kann dabei auch eine präventive Therapiephase sein, die nach einer erfolgreichen ersten Therapiephase erfolgt, um ein Wiederauftreten der Kniegelenkbeschwerden zu verhindern. Insbesondere kann die zweite Therapiephase bei sportlicher Betätigung des Patienten erfolgen, um dabei das Kniegelenk zu stabilisieren und zu entlasten, wodurch Verletzungen oder Überbeanspruchungen verhindert werden können.

Die erfindungsgemäße Kniegelenkbandage stellt eine optimierte Bewegungsführung des Kniegelenks sicher und verhindert einerseits ein Abdriften der Patella nach außen (also in lateraler Richtung) und unterdrückt andererseits ein Hochschieben der Patella in proximaler Richtung. Insbesondere übt die erfindungsgemäße Kniegelenkbandage bei der Therapie von Kniegelenksbeschwerden eine sowohl in medial-lateraler Richtung als auch in proximaldistaler Richtung eine zentrierende Wirkung auf die Patella aus, um diese bei einer Bewegung des Kniegelenks in einer physiologisch optimierten Position zu halten. Die Kniegelenkbandage gemäß der Erfindung ermöglicht daher eine Lateralisierung der Patella und kann sowohl zur Behandlung eines Patellahochstands als auch zur Behandlung der Osgood-Schlatter-Krankheit eingesetzt werden.

Diese und weitere Vorteile sowie Merkmale der Erfindung ergeben sich aus dem nachfolgend unter Bezugnahme auf die begleitenden Zeichnungen näher beschriebenen Ausführungsbeispiel, wobei die Zeichnungen zeigen:
- **Fig. 1**: Vorderansicht auf eine Ausführungsform einer erfindungsgemäßen Kniegelenkbandage;
- **Fig. 2**: perspektivische Ansicht auf die Kniegelenkbandage von Figur 1 in einem an einem Kniegelenk angelegten Zustand;
- **Fig. 3**: Darstellung der Verwendung der Kniegelenkbandage von Figur 1 in einer ersten Therapiephase durch eine Schnittdarstellung der Kniegelenkbandage in einem an einem gebeugten Kniegelenk angelegten Zustand;
- **Fig. 4**: Detailansicht der zweiten Pelotte der Kniegelenkbandage von Figur 1 mit einem zugehörigen Halteband zur Ausbildung einer Patellasehnenbandage zum Anlegen der zweiten Pelotte an einem Kniegelenk;
- **Fig. 5**: Darstellung der Verwendung der Patellasehnenbandage von Figur 4 in einer zweiten Therapiephase durch eine Schnittdarstellung der Patellasehnenbandage in einem an einem gebeugten Kniegelenk angelegten Zustand, wobei die zweite Pelotte mit dem zugehörigen Halteband am Kniegelenk unterhalb der Patella angelegt ist.

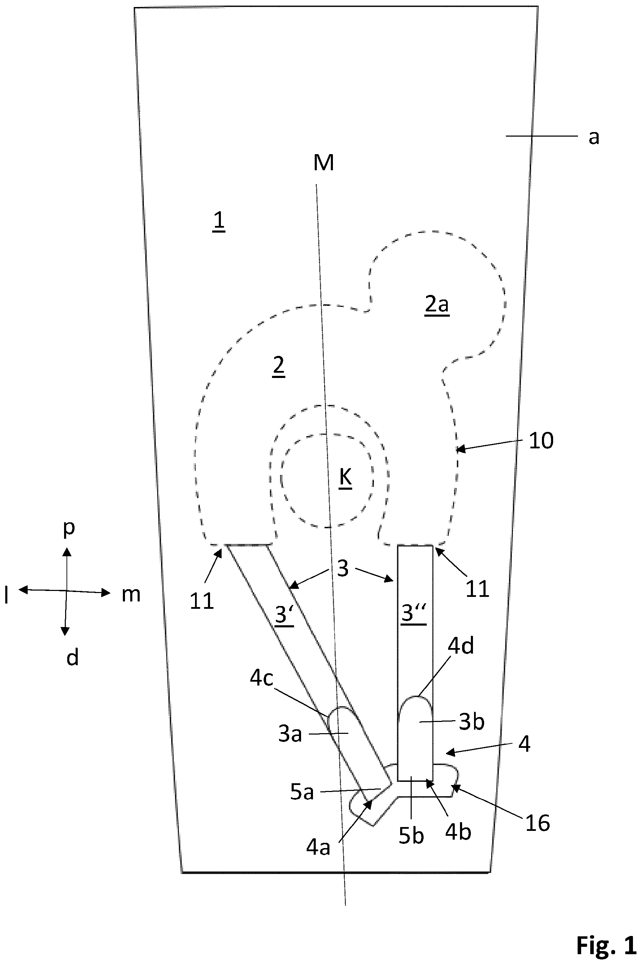

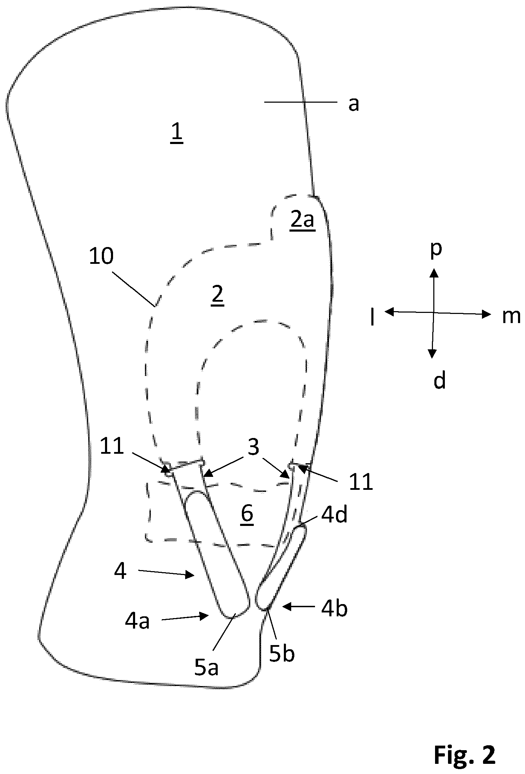

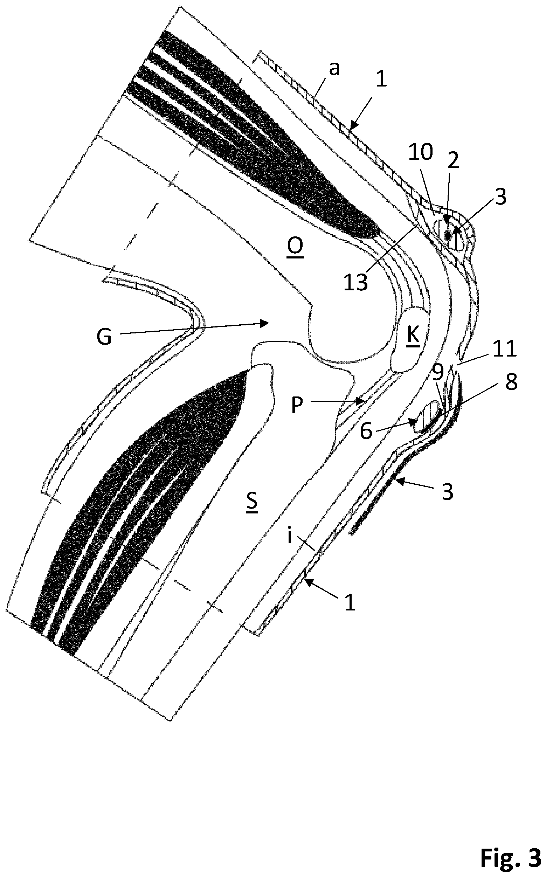

Die in den Figuren 1 und 2 gezeigte Ausführungsform einer erfindungsgemäßen Kniegelenkbandage umfasst einen schlauchförmigen Grundkörper 1 aus einem elastischen Textilmaterial, beispielsweise aus einem elastischen Gestrick, insbesondere aus einem Kompressionsgestrick, welches über einen kompressionsgebenden, elastischen Schussfaden verfügt. Der Grundkörper 1 weist dabei eine Außenseite a und bei an einem Kniegelenk G angelegter Bandage dem Kniegelenk G zugewandten Innenseite i auf, wie in Figur 3 gezeigt. Die Kniegelenkbandage umfasst weiterhin eine am Grundkörper 1 angeordnete und insbesondere in dem Grundkörper 1 integrierte erste Pelotte 2 und ein mit der ersten Pelotte 2 verbundenes Zugband 3. Die erste Pelotte 2 ist aus einem elastischen Material, beispielsweise aus Silikon oder Silikonkautschuk oder einem Gummimaterial gefertigt und ist zumindest im Wesentlichen U-förmig oder hufeisenförmig ausgebildet. Das zugfest mit der ersten Pelotte 2 verbundene Zugband ist aus einem unelastischen Material oder aus einem Material, das zumindest weniger elastisch als das Material der ersten Pelotte 2 ist und weist ein erstes Ende 3a und ein zweites Ende 3b auf. Das Zugband 3 setzt sich aus insgesamt drei Abschnitten 3', 3" und 3‴ zusammen, wobei ein erster Abschnitt 3' und ein zweiter Abschnitt 3" jeweils auf der Außenseite a des Grundkörpers 1 und ein den ersten und dem zweiten Abschnitt verbindender dritter Abschnitt 3‴ im Inneren des schlauchförmigen Grundkörpers 1, also an dessen Innenseite i angeordnet ist. Zum Ein- bzw. Herausführen des Zugbands 3 in das Innere des schlauchförmigen Grundkörpers 1 bzw. aus diesem heraus sind in dem Grundkörper 1 zwei Öffnungen 11, insbesondere in Form von Schlitzen vorgesehen, durch die das Zugband 3 geführt ist. Der im Inneren des schlauchförmigen Grundkörpers 1 verlaufende dritte Abschnitt 3‴ des Zugbands 3 verläuft dabei innerhalb der ersten Pelotte 2. Zweckmäßig ist der dritte Abschnitt 3‴ des Zugbands 3 in das elastische Material der ersten Pelotte 2 eingegossen und dadurch zugfest mit der ersten Pelotte 2 verbunden. Der innere, dritte Abschnitt 3‴ des Zugbands 3 kann jedoch auch außerhalb der ersten Pelotte 2 angeordnet und mit dieser z.B. durch Verkleben zugfest verbunden sein.

Zur Aufnahme der ersten Pelotte 2 und des darin verlaufenden dritten Abschnitts 3‴ des Zugbands 3 ist an der Innenseite i des Grundkörpers 1 eine in den Figuren 1 und 2 gestrichelt dargestellte Tasche 10 vorgesehen. Die Tasche 10 kann beispielsweise an der Innenseite i des Grundkörpers 1 vernäht und auf diese Weise mit dem Grundkörper 1 verbunden sein. Um einen Zugang zur Tasche 10 zu bilden, ist die Tasche 10 mit den Öffnungen 11 im Grundkörper 1 verbunden, so dass der dritte Abschnitt 3‴ des Zugbands 3 durch eine der beiden Öffnungen 11 auf der einen Seite in die Tasche 10 ein- und auf der anderen Seite durch die andere Öffnung 11 aus der Tasche 10 herausgeführt werden kann.

Wie insbesondere aus der Vorderansicht der Figur 1 ersichtlich weist die Tasche 10 und die darin angeordnete erste Pelotte 2 eine Ausbuchtung 2a im medial-proximalen Bereich auf. Diese Ausbuchtung 2a dient dazu eine stimulierende Wirkung auf die Muskulatur auszuüben. Wie weiterhin insbesondere der Vorderansicht der Figur 1 zu entnehmen ist, umgreift die in der Tasche 10 angeordnete erste Pelotte 2 die in Figur 1 gestrichelt dargestellte Patella K im proximalen sowie im medialen und lateralen Bereich. In den Figuren 1 und 2 sind die auf das Kniegelenk G bezogenen Richtungen medial m, lateral l, proximal p und distal d durch Pfeile angegeben.

Die beiden äußeren Abschnitte 3' und 3" des Zugbands 3 sind mittels Fixiermittel 4 festgelegt. Bei dem in den Figuren 1 und 2 gezeigten Ausführungsbeispiel ist sowohl der erste Abschnitt 3', der das erste Ende 3a des Zugbands 3 enthält, und der zweite Abschnitt 3", der das zweite Ende 3b des Zugbands 3 enthält, über jeweils ein zugeordnetes Umlenkmittel 4a, 4b um 180° umgelenkt. Dabei ist sowohl am ersten Ende 3a als auch am zweiten Ende 3b des Zugbands 3 jeweils an Befestigungsmittel 4c, 4d angeordnet, mit dem der jeweils um 180° umgelenkte Abschnitt des Zugbands 3 an dem äußeren Abschnitt 3' bzw. 3" befestigt ist. Bei den Umlenkmitteln 4a, 4b kann es sich beispielsweise um Umlenkösen oder - wie in den Ausführungsbeispielen der Figuren 1 und 2 gezeigt, um Umlenköffnungen in einer auf der Außenseite a des Grundkörpers 1 befestigten Umlenklasche 16 handeln.

Die Position der Umlenkmittel 4a, 4b definiert dabei für jeden der beiden Abschnitte 3' und 3" des Zugbands 3 einen Verankerungspunkt 5a bzw. 5b, an dem der jeweilige Abschnitt 3' bzw. 3" des Zugbands 3 in Bezug auf den Grundkörper 1 verankert ist. So definiert das dem ersten Abschnitt 3' des Zugbands 3 zugeordnete erste Umlenkmittel 4a einen ersten Verankerungspunkt 5a und das dem zweiten Abschnitt 3" zugeordnete zweite Umlenkmittel 4b definiert einen zweiten Verankerungspunkt 5b. Wie insbesondere aus der Vorderansicht der Figur 1 ersichtlich, liegen sowohl der erste Verankerungspunkt 5a als auch der zweite Verankerungspunkt 5b sowohl distal als auch medial zu der Sagittalebene M, die durch den Mittelpunkt der Patella K geht und die Mittellängsachse der Patellasehne P enthält. Die sich unterhalb der Patella K erstreckende Patellasehne P, welche die Patella K mit dem Schienbein S verbindet, erstreckt sich dabei in dieser Sagittalebene M. Damit liegen die beiden Verankerungspunkte 5a, 5b sowohl medial als auch distal zur Patellasehne P.

Die Position der Verankerungspunkte bestimmt dabei zusammen mit der Position der Öffnungen 11, durch die die äußeren Abschnitte 3', 3" des Zugbands 3 nach außen geführt sind, die Lage der äußeren Abschnitte 3' und 3" in Bezug auf den Grundkörper 1, wobei sich die sich die äußeren Abschnitte 3' und 3" jeweils zwischen der zugeordneten Öffnung 11 und dem ersten Verankerungspunkt 5a bzw. dem zweiten Verankerungspunkt 5b erstrecken.

Durch die Anordnung der Verankerungspunkte 5a, 5b des Zugbands 3 kann bei einer Festlegung des Zugbands 3 mittels der Befestigungsmittel 4c, 4d eine Zugkraft auf die zugfest mit dem Zugband 3 verbundene erste Pelotte 2 ausgeübt werden, wobei diese Zugkraft im Wesentlichen in distaler Richtung gerichtet ist und aufgrund der asymmetrischen Anordnung der beiden Verankerungspunkte 5a, 5 b bezüglich der Sagitallebene M bzw. in Bezug auf die Patellesehne P auch eine Kraftkomponente in medialer Richtung aufweist. Die Richtungen der von den beiden äußeren Abschnitten 3' und 3" des Zugbands 3 auf die erste Pelotte 2 ausgeübten Zugkräfte ergeben sich durch die Lage der beiden äußeren Abschnitte 3' und 3" des Zugbands 3, die dem Ausführungsbeispiel der Figur 1 zu entnehmen ist. In diesem Ausführungsbeispiel schließen die beiden äußeren Abschnitte 3' und 3" des Zugbands 3 miteinander einen Winkel von ca. 45° ein.

Bei einer Bewegung des Kniegelenks, insbesondere bei einer Beugung des Knies, werden die beiden Verankerungspunkte 5a, 5b, an denen das Zugband 3 auf der Außenseite a des Grundkörpers 1 verankert ist, in distaler Richtung nach unten bewegt, wodurch die beiden außenliegenden Abschnitte 3' und 3" des Zugbands 3 eine in der jeweiligen Längsrichtung des ersten bzw. des zweiten Abschnitts 3', 3" gerichtete Zugkraft auf die erste Pelotte 2 ausüben. Durch den in distal-proximaler Richtung verlaufenden zweiten Abschnitt 3" des Zugbands 3 wird auf die erste Pelotte 2 eine Kraft in distaler Richtung ausgeübt. Aufgrund der asymmetrischen Anordnung der beiden Verankerungspunkte 5a, 5b in Bezug auf die Sagitallebene M bzw. in Bezug auf die Patellasehne P und die damit verbundene Schrägstellung des ersten Abschnitts 3' des Zugbands 3 (wie aus Figur 1 ersichtlich) wirkt auf die erste Pelotte 2 weiterhin eine durch den ersten Abschnitt 3' des Zugbands 3 ausgeübte Zugkraft, welche eine Kraftkomponente in distaler Richtung und eine Kraftkomponente in medialer Richtung umfasst. Insgesamt wirkt durch das Zugband 3 auf die erste Pelotte 2 damit eine in distaler und medialer Richtung weisende Kraft ein, wodurch bei einer Bewegung des Kniegelenks, insbesondere bei einer Beugung des Knies, eine Zentrierung der Patella K in ihre physiologisch korrekte Stellung erfolgt, indem die vom Zugband 3 auf die erste Pelotte 2 übertragene Zugkraft in distaler und medialer Richtung auf die Patella K einwirkt, welche in ihrem proximalen sowie medial-lateralen Bereich von der ersten Pelotte 2 umgeben ist. Dadurch erfolgt bei einer Bewegung des Kniegelenks durch die vom Zugband 3 auf die erste Pelotte 2 ausgebübte Zugkraft einerseits eine Lateralisierung der Patella K und andererseits wird ein Hochschieben der Patella K in proximaler Richtung unterdrückt.

Dieser Mechanismus ist in der Schnittdarstellung der Figur 3 zu erkennen, in der die Verwendung der Kniegelenkbandage in einer ersten Therapiephase durch eine Schnittdarstellung der Kniegelenkbandage in einem an einem gebeugten Kniegelenk angelegten Zustand gezeigt ist. Dabei ist die Kniegelenkbandage so am Kniegelenk G angelegt, dass die erste Pelotte 2 mit dem darin verlaufenden dritten Abschnitt 3‴ des Zugbands 3 die Patella K in ihrem proximalen sowie im lateralen und medialen Bereich umgreift.

In einer bevorzugten Ausführungsform, welche in der Schnittdarstellung der Figur 3 verdeutlicht ist, umfasst die Kniegelenkbandage neben der ersten Pelotte 2, die an der Innenseite i des Grundkörpers 1 in der Tasche 10 angeordnet ist, noch eine zweite Pelotte 6, welche ebenfalls an der Innenseite i des Grundkörpers 1 befestigt und distal zur Patella K angeordnet ist. In der Funktionsdarstellung der Figur 2 ist die Lage der zweiten Pelotte 6 im Bereich unterhalb der ersten Pelotte 2 gestrichelt dargestellt. Die zweite Pelotte 6 ist dabei bandförmig ausgebildet und verläuft bei am Kniegelenk G angelegter Kniegelenkbandage quer über die Patellasehne P, wie aus Figur 3 ersichtlich.

Die dadurch als Patellasehnenbandage wirkende zweite Pelotte 6 ist dabei bevorzugt abnehmbar an der Innenseite i des Grundkörpers 1 befestigt, beispielsweise durch zusammenwirkende Befestigungselemente 8, 9. Bei den Befestigungselementen 8, 9 kann es sich beispielsweise um die Elemente eines Klettverschlusses, insbesondere um ein Hakenteil und ein zugehöriges Flauschteils eines Klettverschlusses handelt. Bevorzugt ist an der dem Kniegelenk G abgewandten Außenseite der zweiten Pelotte 6 ein Befestigungselement 8 in Form eines Hakenteils eines Klettverschlusses angeordnet, welches mit einem dazu korrespondierenden Flauschteil an der Innenseite i des Grundkörpers 1 zusammenwirkt, um die bandförmige zweite Pelotte 6 abnehmbar an der Innenseite i des Grundkörpers 1 zu befestigen.

Wenn die zweite Pelotte 6 an der aus Figur 3 ersichtlichen Position an der Innenseite i des Grundkörpers 1 befestigt ist, übt die zweite Pelotte 6 einen Druck auf die Patellasehne P aus und setzt diese damit bei einer Bewegung des Kniegelenks unter Spannung. Dadurch werden die Sehnen, Knochenansätze und Knorpelstrukturen im Kniegelenk entlastet. Die zweite Pelotte 6 wirkt dadurch stabilisierend und entlastend auf das Kniegelenk und wirkt Bewegungseinschränkungen bei einer Degeneration des Kniegelenks entgegen.

Gleichzeitig wird bei einer Bewegung des Kniegelenks G, insbesondere beim Beugen des Knies, ein Hochschieben der Patella K in proximaler Richtung, welches durch den von der zweiten Pelotte 6 ausgeübten Druck auf die Patellasehne P verursacht werden kann, durch die proximal der Patella K angeordnete und diese im proximalen sowie im medial-lateralen Bereich umgebende erste Pelotte 2 verhindert. Damit wird die Patella K bei angelegter Kniegelenkbandage bei einer Beugung des Knies in ihrer physiologisch korrekten Lage zentriert, ohne dass es zu einem Hochschieben der Patella K in proximaler Richtung (nach oben) und zu einem Abdriften in lateraler Richtung (nach außen) kommen kann.

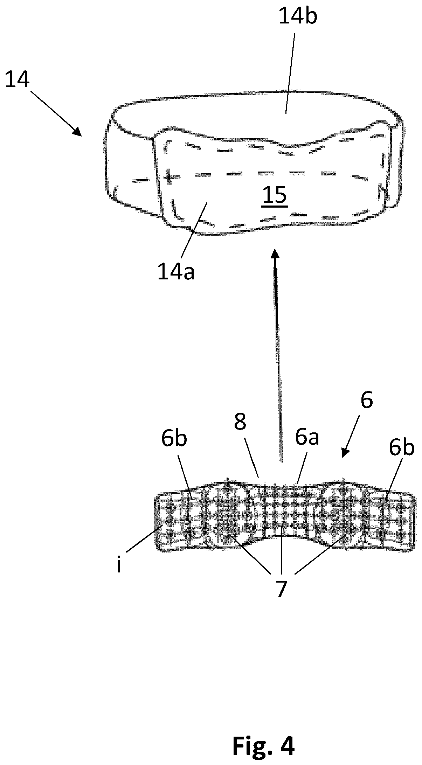

Die in Figur 4 unten im Detail gezeigte zweite Pelotte 6 ist zumindest im Wesentlichen bandförmig ausgebildet und weist eine Längsseite auf, die sich bei angelegter Kniegelenkbandage quer über die Patellasehne P erstreckt, wenn die zweite Pelotte 6, wie in Figur 3 gezeigt, an der Innenseite i des Grundkörpers 1 befestigt ist. Die zweite Pelotte 6 weist dabei zweckmäßig einen mittleren Abschnitt 6a sowie zwei sich seitlich daran anschließende randseitige Abschnitte 6b auf, wobei die randseitigen Abschnitte 6b vom mittleren Abschnitt 6a nach unten, d.h., in distaler Richtung, abgekröpft sind, wie aus Figur 4 unten ersichtlich. Unter abgekröpft wird dabei verstanden, dass die randseitigen Abschnitte 6b winklig unter einem Winkel im Bereich von 10° bis 30° zum mittleren Abschnitt 6a verlaufen. Die distal abgekröpften randseitigen Abschnitte 6b ermöglichen eine bessere Passform und Anschmiegsamkeit über die Hoffa'schen Fettkörper.

Zur Erzielung eines Massageeffekts bei angelegter zweiter Pelotte 6 weist die bei angelegter Kniebandage dem Kniegelenk zugewandte Innenseite der zweiten Pelotte 6 eine Mehrzahl von Stimulationselementen auf, insbesondere in Form von Erhebungen oder Vorsprüngen und bevorzugt in Form von Noppen.

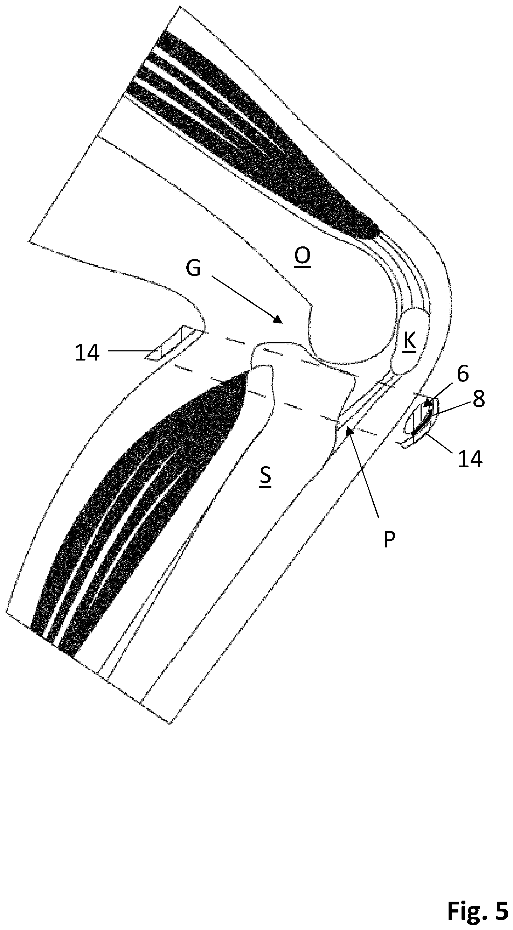

Die in Figur 4 unten gezeigte zweite Pelotte 6 kann in einer gesonderten zweiten Therapiephase auch ohne den Grundkörper 1 mit der darin integrierten ersten Pelotte 2 verwendet werden. Hierzu ist ein in Figur 4 oben gezeigtes Halteband 14 vorgesehen, welches über einen Aufnahmeabschnitt 14a verfügt, an dem die zweite Pelotte 6 abnehmbar befestigt werden kann. Zweckmäßig weist der Aufnahmeabschnitt 14a lösbare Befestigungsmittel 15 auf, insbesondere einen Teil eines Klettverschlusses, mit denen die zweite Pelotte 6 am Aufnahmeabschnitt 14a des Haltebands 14 befestigt werden kann. Wenn auf der einen Seite (Außenseite) der zweiten Pelotte 6 ein Hakenteil eines Klettverschlusses angeordnet ist, mit dem die zweite Pelotte 6 an der Innenseite i des Grundkörpers 1 befestigbar ist (wie in Figur 3 gezeigt), sind die Befestigungsmittel 15 am Aufnahmeabschnitt 14a des Haltebands 14 zweckmäßig als dazu korrespondierendes Flauschteil eines Klettverschlusses ausgebildet. Zur Befestigung des Haltebands 14 am Kniegelenk G weist das Halteband 14 einen mit dem Aufnahmeabschnitt 14a verbundenen Halteabschnitt 14b auf, wobei der Halteabschnitt 14b bevorzugt als längenverstellbarer Gurt oder als elastisches Band, beispielsweise als Gummiband, ausgebildet ist. Über den längenverstellbaren bzw. elastischen Halteabschnitt 14b kann das Halteband 14 mit der am Aufnahmeabschnitt 14a befestigten zweiten Pelotte 6 am Kniegelenk G angelegt werden, wie in Figur 5 gezeigt. Dabei verläuft das Halteband 14 im Bereich unterhalb der Kniekehle und erstreckt sich über den Umfang des oberen Abschnitts des Unterschenkels, wobei die an der Innenseite am Aufnahmeabschnitt 14a des Haltebands 14 befestigte zweite Pelotte 6 im Bereich der Patellasehne P liegt und sich quer über die Patellasehne P erstreckt. In dieser Position der zweiten Pelotte 6 übt diese insbesondere bei einer Bewegung des Kniegelenks G einen Druck auf die Patellasehne P aus. Durch die Stimulationselemente 7 wird die Patellasehne P und das diese umgebende Gewebe stimuliert, wodurch die Durchblutung des Körpergewebes verbessert wird.

Die erfindungsgemäße Kniegelenkbandage kann damit einerseits in der in Figur 3 gezeigten Weise und andererseits in der in Figur 5 gezeigten Weise zur Therapie von Kniegelenksbeschwerden verwendet werden, wobei in der in Figur 3 gezeigten ersten Verwendungsform sowohl die erste Pelotte 2 als auch die zweite Pelotte 6 einen Druck auf bestimmte Bereiche des Kniegelenks G ausübt und in der in Figur 5 gezeigten zweiten Verwendungsform lediglich durch die zweite Pelotte 6 ein Druck auf die Patellasehne P ausgeübt wird.

Die erfindungsgemäße Kniegelenkbandage kann daher zur Behandlung von unterschiedlichen Kniebeschwerden, wie z.B. bei Schädigungen des Kniegelenks im Gleitlager zwischen Patella und Oberschenkel, bei einem Patellahochstand, beim femoropatellaren Schmerzsyndrom, bei Patella-Luxationen oder -Subluxationen, bei Morbus Osgood Schlatter, bei einer Gonararthrose oder bei Gelenkergüssen und Reizzuständen im Kniegelenk einerseits therapierend und anderesseits auch präventiv eingesetzt werden. Insbesondere kann eine kombinierte Therapie mit einer Verwendung der erfindungsgemäßen Kniegelenkbandage in der in Figur 3 gezeigten Verwendungsform in einer ersten Therapiephase und danach in einer zweiten Therapiephase in der in Figur 5 gezeigten Verwendungsform zielführend ist. In der ersten Therapiephase kann die Bandage bspw. mit an der Innenseite befestigter zweiter Pelotte 6 bei einer Therapiedauer von 10 bis 100 Tagen eingesetzt werden. An diese erste Therapiephase kann sich die zweite Therapiephase anschließen, in der die zweite Pelotte 6 zusammen mit dem zugehörigen Halteband 14 als Patellasehnenbandage wie aus Figur 3 ersichtlich am Kniegelenk angelegt wird. Die zweite Therapiephase kann dabei auch eine präventive Phase sein, in der die Patellasehnenbandage bspw. zur Prävention nur während sportlicher Betätigung des Patienten eingesetzt wird.

Die Erfindung ist nicht auf das hier zeichnerisch dargestellte Ausführungsbeispiel beschränkt. So kann beispielsweise auf die Umlenkung der beiden äußeren Abschnitte 3', 3" des Zugbands 3 verzichtet werden. Anstelle der zum Umlenken der Abschnitte 3', 3" des Zugbands 3 eingesetzten Umlenkmittel 4a, 4b können die beiden Enden 3a, 3b des Zugbands 3 auch unmittelbar ohne Umlenkung mittels der an den Enden 3a, 3b angeordneten Befestigungsmittel 4c, 4c an der Außenseite a des Grundköpers 1 befestigt werden. In diesem, hier zeichnerisch nicht dargestellten Ausführungsbeispiel der erfindungsgemäßen Kniegelenkbandage werden die Verankerungspunkte 5a, 5b des Zugbands 3 durch die Position der Befestigungsmittel 4c, 4d definiert, durch welche die Enden 3a, 3b des Zugbands 3 am Grundkörper 1 befestigt sind. Maßgeblich für die Definition der beiden Verankerungspunkte 5a, 5b ist dabei die Stelle, an der die Abschnitte 3', 3" des Zugbands 3 am Grundkörper 1 verankert oder fixiert sind.

Weiterhin können sowohl die erste Pelotte 2 als auch die zweite Pelotte 6 anders ausgeformt sein. Insbesondere können an der ersten Pelotte 2 noch weitere Ausbuchtungen oder zusätzliche Stimulationselemente vorgesehen sein, welche insbesondere zu einer interplanetaren Stimulation des Kniegelenks G beitragen.

Weiterhin kann einer der beiden Verankerungspunkte distal der Patella K liegend auch in der Linie der Verlängerung der Patellasehne P in deren Längsrichtung auf dem Schienbein S liegen. Auch in diesem Fall ist eine asymmetrische Anordnung der beiden Verankerungspunkte 5a, 5b in Bezug auf die Sagittalebene M bzw. bezüglich der Patellasehne P gegeben, die eine Kraftkomponente der vom Zugband 3 auf die erste Pelotte 3 übertragenen Zugkraft in medialer Richtung erzeugt.

Die erfindungsgemäße Kniegelenkbandage kann weiterhin zusätzliche Stabilisierungselemente aufweisen, die insbesondere im oder am Grundkörper angeordnet sein können, wie z.B. Stabilisierungsstangen oder -stäbe aus Metall oder Kunststoff, die insbesondere seitlich am Grundkörper und entlang dessen Längsrichtung von proximal nach distal verlaufen können. Da Bandagen mit derartigen Stabilisierungselementen häufig auch als Orthesen bezeichnet werden, sind die Begriffe Bandage und Orthese hier synonym zu verstehen.

## Patentansprüche

1. Kniegelenkbandage umfassend einen schlauchförmigen Grundkörper (1) aus einem elastischen Textilmaterial, zumindest eine am Grundkörper angeordnete erste Pelotte (2) und ein mit der Pelotte (2) verbundenes Zugband (3) mit einem ersten Ende (3a) und einem zweiten Ende (3b), wobei die erste Pelotte (2) bei an einem Kniegelenk (G) angelegter Kniegelenkbandage proximal zur Kniescheibe (K) positioniert ist und die Kniescheibe (K) zumindest teilweise umgreift und die beiden Enden (3a, 3b) des Zugbands (3) distal der Kniescheibe am Grundkörper (1) mittels lösbarer Fixiermittel (4) verankerbar sind, wobei das erste Ende (3a) des Zugbands (3) an einem vorgegebenen ersten Verankerungspunkt (5a) und das zweite Ende (3b) des Zugbands (3) an einem vorgegebenen zweiten Verankerungspunkt (5b) verankerbar ist, wobei bei angelegter Kniegelenkbandage beide Verankerungspunkte (5a, 5b) jeweils distal der Kniescheibe (K) liegen, **dadurch gekennzeichnet, dass** gleichzeitig entweder beide Verankerungspunkte (5a, 5b) medial zur Patellasehne (P) liegen oder einer der beiden Verankerungspunkte (5b) medial zur Patellasehne (P) und der andere Verankerungspunkt (5a) distal zur Patellasehne (P) in der Linie der Verlängerung der Patellasehne (P) in Längsrichtung auf dem Schienbein (S) liegt.

2. Kniegelenkbandage nach Anspruch 1, **dadurch gekennzeichnet, dass** das Zugband (3) durchgehend oder abschnittsweise an Unterbrechungsstellen unterbrochen ist, wobei das durchgehende Zugband (3) durch die erste Pelotte (2) verläuft und das abschnittsweise unterbrochene Zugband (3) an den Unterbrechungsstellen an der ersten Pelotte (2) befestigt ist.

3. Kniegelenkbandage nach einem der voranstehenden Ansprüche, **dadurch gekennzeichnet, dass** die Fixiermittel (4) für jedes Ende (3a, 3b) des Zugbands (3) Umlenkmittel (4a, 4b) umfassen, welche das Zugband (3) nahe des jeweiligen Endes (3a, 3b) umlenken.

4. Kniegelenkbandage nach einem der voranstehenden Ansprüche, **dadurch gekennzeichnet, dass** die Fixiermittel (4) Befestigungsmittel (4c, 4d) umfassen, wobei an jedem Ende (3a, 3b) des Zugbands (3) ein Befestigungsmittel (4c, 4d) angeordnet ist, mit dem das jeweilige Ende (3a, 3b) des Zugbands (3) lösbar an einem Abschnitt (3', 3") des Zugbands (3) oder auf der Außenseite des Grundkörpers (1) befestigbar ist.

5. Kniegelenkbandage nach einem der voranstehenden Ansprüche, **dadurch gekennzeichnet, dass** bei angelegter Kniegelenkbandage der erste Verankerungspunkt (5a) medial zur Mittellängsachse des Schienbeins (S) liegt und der zweite Verankerungspunkt (5b) ebenfalls medial zur oder auf der Mittellängsachse des Schienbeins (S) liegt.

6. Kniegelenkbandage nach einem der voranstehenden Ansprüche, **dadurch gekennzeichnet, dass** die erste Pelotte (2) zumindest im Wesentlichen U-förmig oder hufeisenförmig ausgebildet ist und bei angelegter Kniegelenkbandage zumindest den proximalen Bereich der Kniescheibe (K) umgibt.

7. Kniegelenkbandage nach Anspruch 6, **dadurch gekennzeichnet, dass** die erste Pelotte (2) im medial-proximalen Bereich eine Ausbuchtung (2a) aufweist, wobei an der Ausbuchtung bevorzugt Stimulationselemente angeordnet sind.

8. Kniegelenkbandage nach einem der voranstehenden Ansprüche, **dadurch gekennzeichnet, dass** distal zur ersten Pelotte (2) eine zweite Pelotte (6) vorgesehen ist, wobei die zweite Pelotte (6) bei angelegter Kniegelenkbandage über die Patellasehne (P) verläuft, wobei die zweite Pelotte (6) bevorzugt abnehmbar an der dem Kniegelenk zugewandten Innenseite des Grundkörpers (1) befestigt ist.

9. Kniegelenkbandage nach Anspruch 8, **dadurch gekennzeichnet, dass** die zweite Pelotte (6) an ihrer zum Kniegelenk zugewandten Innenseite Stimulationselemente (7), bevorzugt in Form einer Mehrzahl von Erhebungen oder Vorsprüngen, insbesondere in Form von Noppen, aufweist und/oder dass die zweite Pelotte (6) an ihrer zum Kniegelenk abgewandten Außenseite Befestigungselemente (8) zur lösbaren Befestigung an der Innenseite des Grundkörpers (1) aufweist.

10. Kniegelenkbandage nach einem der Ansprüche 8 bis 9, **dadurch gekennzeichnet, dass** die zweite Pelotte (6) zumindest im Wesentlichen bandförmig mit einer Längsseite ausgebildet ist, wobei die zweite Pelotte (6) so an der Innenseite des Grundkörpers (1) befestigt ist, dass sie bei angelegter Kniegelenkbandage mit ihrer Längsseite quer über die Patellasehne (P) verläuft, wobei die zweite Pelotte (6) insbesondere einen bei angelegter Kniegelenkbandage quer über die Patellasehne (P) verlaufenden mittleren Abschnitt (6a) sowie zwei sich daran anschließende randseitige Abschnitte (6b) aufweist, wobei die randseitigen Abschnitte (6b) vom mittleren Abschnitt (6a) bevorzugt nach distal abgekröpft sind.

11. Kniegelenkbandage nach einem der Ansprüche 8 bis 10, **dadurch gekennzeichnet, dass** an der Innenseite (i) des Grundkörpers (1) ein Befestigungsmittel (9), insbesondere ein Teil eines Klettverschlusses, zur lösbaren Befestigung der zweiten Pelotte (6) und/oder eine Tasche (10) zur Aufnahme der ersten Pelotte (2) angeordnet ist.

12. Kniegelenkbandage nach einem der voranstehenden Ansprüche, **dadurch gekennzeichnet, dass** das Zugband (3) einen ersten Abschnitt (3') und einen zweiten Abschnitt (3") umfasst, wobei der erste Abschnitt (3') das erste Ende (3a) und der zweite Abschnitt (3") das zweite Ende (3b) des Zugbands (3) enthält.

13. Kniegelenkbandage nach Anspruch 12, **dadurch gekennzeichnet, dass** das Zugband (3) einen dritten Abschnitt (3‴) umfasst, der den ersten Abschnitt (3') und den zweiten Abschnitt (3") verbindet und in einer an der Innenseite des Grundkörpers (1) angeordneten Tasche (10) verläuft.

14. Kniegelenkbandage nach Anspruch 12 oder 13, **dadurch gekennzeichnet, dass** im Grundkörper (1) wenigstens zwei mit der Tasche (10) in Verbindung stehende Schlitze (11) eingearbeitet sind, durch welche das Zugband (3) in die Tasche (10) ein- bzw. aus der Tasche (10) herausgeführt ist, wobei die Schlitze (11) horizontal und/oder schräg zur Horizontalen verlaufend ausgeführt sind.

## Claims

1. Knee joint bandage comprising a tubular base body (1) made of an elastic textile material, at least one first pad (2) arranged on the base body and a tension band (3) connected to the pad (2) and having a first end (3a) and a second end (3b), wherein the first pad (2) is positioned proximal to the kneecap (K) when the knee joint bandage is applied to a knee joint (G) and at least partially embraces the kneecap (K) and the two ends (3a, 3b) of the tension band (3) can be anchored distally of the kneecap to the base body (1) by means of releasable fixing means (4), wherein the first end (3a) of the tension band (3) can be anchored at a predetermined first anchoring point (5a) and the second end (3b) of the tension band (3) can be anchored at a predetermined second anchoring point (5b), wherein, when the knee joint bandage is applied, both anchoring points (5a, 5b) lie distal to the kneecap (K), **characterised in that in that** at the same time either both anchoring points (5a, 5b) lie medial to the patella tendon (P) or one of the two anchoring points (5b) lies medial to the patella tendon (P) and the other anchoring point (5a) lies distal to the patella tendon (P) in the line of extension of the patella tendon (P) in the longitudinal direction on the tibia (S).

2. Knee joint bandage according to claim 1, **characterised in that** the tension band (3) is continuous or is sectionally interrupted at interruption points, wherein the continuous tension band (3) is running through the first pad (2) and the sectionally interrupted tension band (3) is fastened to the first pad (2) at the interruption points.

3. Knee joint bandage according to one of the preceding claims, **characterised in that** the fixing means (4) for each end (3a, 3b) of the tension band (3) comprise deflection means (4a, 4b) which deflect the tension band (3) close to the respective end (3a, 3b).

4. Knee joint bandage according to one of the preceding claims, **characterised in that** the fixing means (4) comprise fastening means (4c, 4d), wherein a fastening means (4c, 4d) is arranged at each end (3a, 3b) of the tension band (3), with which the respective end (3a, 3b) of the tension band (3) can be detachably fastened to a portion (3', 3") of the tension band (3) or to the outside of the base body (1).

5. Knee joint bandage according to one of the preceding claims, **characterised in that**, when the knee joint bandage is applied, the first anchoring point (5a) lies medial to the central longitudinal axis of the tibia (S) and the second anchoring point (5b) also lies medial to or on the central longitudinal axis of the tibia (S).

6. Knee joint bandage according to one of the preceding claims, **characterised in that** the first pad (2) is at least substantially U-shaped or horseshoe-shaped and surrounds at least the proximal region of the kneecap (K) when the knee joint bandage is applied.

7. Knee joint bandage according to claim 6, **characterised in that** the first pad (2) has a bulge (2a) in the medial-proximal region, wherein stimulation elements are preferably arranged on the bulge.

8. Knee joint bandage according to one of the preceding claims, **characterised in that** a second pad (6) is provided distally to the first pad (2), wherein the second pad (6) extends over the patella tendon (P) when the knee joint bandage is applied, wherein the second pad (6) is preferably detachably fastened to the inner side of the base body (1) facing the knee joint.

9. Knee joint bandage according to claim 8, **characterised in that** the second pad (6) has stimulation elements (7), preferably in the form of a plurality of elevations or projections, in particular in the form of knobs, on its inner side facing the knee joint, and/or **in that** the second pad (6) has fastening elements (8) on its outer side facing away from the knee joint for detachably fastening to the inner side of the base body (1).

10. Knee joint bandage according to one of the claims 8 to 9, **characterised in that** the second pad (6) is formed at least substantially in the shape of a band with a longitudinal side, the second pad (6) being fastened to the inner side of the base body (1) in such a way that, when the knee joint bandage is applied, it runs with its longitudinal side transversely over the patella tendon (P), wherein the second pad (6) in particular has a central section (6a) extending transversely over the patella tendon (P) when the knee joint bandage is applied and two adjoining edge sections (6b), wherein the edge sections (6b) are preferably offset distally from the central section (6a).

11. Knee joint bandage according to one of the claims 8 to 10, **characterised in that** a fastening means (9), in particular a part of a Velcro fastener, for detachably fastening the second pad (6) and/or a pocket (10) for receiving the first pad (2) is arranged on the inner side (i) of the base body (1).

12. Knee joint bandage according to one of the preceding claims, **characterised in that** the tension band (3) comprises a first portion (3') and a second portion (3"), the first portion (3') including the first end (3a) and the second portion (3") including the second end (3b) of the tension band (3).

13. Knee joint bandage according to claim 12, **characterised in that** the tension band (3) comprises a third portion (3") which connects the first portion (3') and the second portion (3") and runs in a pocket (10) arranged on the inside of the base body (1).

14. Knee joint bandage according to claim 12 or 13, **characterised in that** at least two slits (11), which are connected to the pocket (10) and through which the tension band (3) is guided into or out of the pocket (10), are incorporated in the base body (1), the slits (11) being designed to run horizontally and/or obliquely to the horizontal.

## Revendications

1. Genouillère comprenant un corps de base (1) en forme de tuyau flexible composé d'un matériau textile élastique, au moins une première pelote (2) disposée sur le corps de base et une bande de traction (3) reliée à la pelote (2) avec une première extrémité (3a) et une seconde extrémité (3b), dans laquelle la première pelote (2) est positionnée de manière proximale par rapport à la rotule (K) lorsque la genouillère est mise en place sur une articulation du genou (G) et entoure au moins en partie la rotule (K) et les deux extrémités (3a, 3b) de la bande de traction (3) peuvent être ancrées du côté distal de la rotule sur le corps de base (1) au moyen de moyens de blocage (4) amovibles, dans laquelle
la première extrémité (3a) de la bande de traction (3) peut être ancrée sur un premier point d'ancrage (5a) spécifié et la seconde extrémité (3b) de la bande de traction (3) peut être ancrée sur un deuxième point d'ancrage (5b) spécifié, dans lequel les deux points d'ancrage (5a, 5b) se situent respectivement du côté distal de la rotule (K) lorsque la genouillère est mise en place,
**caractérisée en ce que**
simultanément soit les deux points d'ancrage (5a, 5b) se situent de manière médiale par rapport au tendon patellaire (P) soit un des deux points d'ancrage (5b) se situe de manière médiale par rapport au tendon patellaire (P) et l'autre point d'ancrage (5a) se situe de manière distale par rapport au tendon patellaire (P) dans la ligne du prolongement du tendon patellaire (P) dans le sens longitudinal sur le tibia (S).

2. Genouillère selon la revendication 1, **caractérisée en ce que** la bande de traction (3) est interrompue en continu ou par endroits sur des points d'interruption, dans laquelle la bande de traction (3) continue s'étend à travers la première pelote (2) et la bande de traction (3) interrompue par endroits est fixée sur la première pelote (2) sur les points d'interruption.

3. Genouillère selon l'une quelconque des revendications précédentes, **caractérisée en ce que** les moyens de blocage (4) comprennent pour chaque extrémité (3a, 3b) de la bande de traction (3) des moyens de renvoi (4a, 4b), qui renvoient la bande de traction (3) à proximité de l'extrémité (3a, 3b) respective.

4. Genouillère selon l'une quelconque des revendications précédentes, **caractérisée en ce que** les moyens de blocage (4) comprennent des moyens de fixation (4c, 4d), dans laquelle est disposé sur chaque extrémité (3a, 3b) de la bande de traction (3) un moyen de fixation (4c, 4d), avec lequel l'extrémité (3a, 3b) respective de la bande de traction (3) peut être fixée de manière amovible sur une section (3', 3'') de la bande de traction (3) ou sur le côté extérieur du corps de base (1) .

5. Genouillère selon l'une quelconque des revendications précédentes, **caractérisée en ce que** le premier point d'ancrage (5a) se situe de manière médiale par rapport à l'axe longitudinal médian du tibia (S) lorsque la genouillère est mise en place et le deuxième point d'ancrage (5b) se situe également de manière médiale par rapport à ou sur l'axe longitudinal médian du tibia (S).

6. Genouillère selon l'une quelconque des revendications précédentes, **caractérisée en ce que** la première pelote (2) est réalisée au moins sensiblement en forme de U ou en forme de fer à cheval et entoure au moins la zone proximale de la rotule (K) lorsque la genouillère est mise en place.

7. Genouillère selon la revendication 6, **caractérisée en ce que** la première pelote (2) présente dans la zone médiale-proximale un renflement convexe (2a), dans laquelle de manière préférée des éléments de stimulation sont disposés sur le renflement convexe.

8. Genouillère selon l'une quelconque des revendications précédentes, **caractérisée en ce qu'**une deuxième pelote (6) est prévue de manière distale par rapport à la première pelote (2), dans laquelle la deuxième pelote (6) s'étend au-dessus du tendon patellaire (P) lorsque la genouillère est mise en place, dans laquelle la deuxième pelote (6) est fixée de manière préférée de manière retirable sur le côté intérieur, tourné vers la genouillère, du corps de base (1).

9. Genouillère selon la revendication 8, **caractérisée en ce que** la deuxième pelote (6) présente, sur son côté intérieur tourné vers l'articulation du genou, des éléments de stimulation (7), de manière préférée sous la forme d'une multitude de parties surélevées ou de parties faisant saillie, en particulier sous la forme de tétons, et/ou que la deuxième pelote (6) présente, sur son côté extérieur opposé à l'articulation du genou, des éléments de fixation (8) destinés à être fixés de manière amovible sur le côté intérieur du corps de base (1).

10. Genouillère selon l'une quelconque des revendications 8 à 9, **caractérisée en ce que** la deuxième pelote (6) est réalisée au moins sensiblement en forme de bande avec un côté longitudinal, dans laquelle la deuxième pelote (6) est fixée de telle sorte sur le côté intérieur du corps de base (1) qu'elle s'étend de manière transversale au-dessus du tendon patellaire (P) par son côté longitudinal lorsque la genouillère est mise en place, dans laquelle la deuxième pelote (6) présente en particulier une section centrale (6a) s'étendant de manière transversale au-dessus du tendon patellaire (P) lorsque la genouillère est mise en place ainsi que deux sections côté bord (6b) s'y raccordant, dans laquelle les sections côté bord (6b) sont coudées depuis la section centrale (6a) de manière préférée vers le côté distal.

11. Genouillère selon l'une quelconque des revendications 8 à 10, **caractérisée en ce qu'**un moyen de fixation (9), en particulier une partie d'une fermeture Velcro, pour la fixation amovible de la deuxième pelote (6) et/ou une poche (10) pour recevoir la première pelote (2) sont disposés sur le côté intérieur (i) du corps de base (1).

12. Genouillère selon l'une quelconque des revendications précédentes, **caractérisée en ce que** la bande de traction (3) comprend une première section (3') et une deuxième section (3"), dans laquelle la première section (3') contient la première extrémité (3a) et la deuxième section (3") contient la seconde extrémité (3b) de la bande de traction (3).

13. Genouillère selon la revendication 12, **caractérisée en ce que** la bande de traction (3) comprend une troisième section (3‴), qui relie la première section (3') et la deuxième section (3'') et s'étend dans une poche (10) disposée sur le côté intérieur du corps de base (1).

14. Genouillère selon la revendication 12 ou 13, **caractérisée en ce que** sont pratiquées dans le corps de base (1) au moins deux entailles (11) reliées à la poche (10), par lesquelles la bande de traction (3) est introduite dans la poche (10) ou est sortie de la poche (10), dans laquelle les entailles (11) sont réalisées de manière à s'étendre horizontalement et/ou à l'oblique par rapport à l'horizontale.
